(19) Europäisches Patentamt
European Patent Office
Office européen des brevets

(11) Publication number : 0 345 995 B1

(12) # EUROPEAN PATENT SPECIFICATION

(45) Date of publication of patent specification : **09.12.92 Bulletin 92/50**

(51) Int. Cl.$^5$ : **B01J 21/06,** B01J 27/135, B01J 27/18, C07C 209/16

(21) Application number : **89305497.3**

(22) Date of filing : **31.05.89**

(54) **Catalytic process for the preparation of linear polyethylenepolyamines with fluorophosphoric acid-on-titania catalysts.**

(30) Priority : **09.06.88 US 204330**

(43) Date of publication of application : **13.12.89 Bulletin 89/50**

(45) Publication of the grant of the patent : **09.12.92 Bulletin 92/50**

(84) Designated Contracting States : **BE DE FR GB IT NL SE**

(56) References cited :
**EP-A- 0 115 138**
**EP-A- 0 197 611**
**EP-A- 0 261 773**
**US-A- 4 698 427**

(73) Proprietor : **TEXACO DEVELOPMENT CORPORATION**
**2000 Westchester Avenue**
**White Plains, New York 10650 (US)**

(72) Inventor : **Grice, Neal John**
**2403 Akron Cove**
**Austin Texas 78723 (US)**

(74) Representative : **Brock, Peter William et al**
**URQUHART-DYKES & LORD 91 Wimpole Street**
**London W1M 8AH (GB)**

EP 0 345 995 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to a process for the preparation of predominantly linear polyethylenepolyamines from ethylenediamine and monoethanolamine in the presence of unique catalyst compositions prepared by depositing a minor amount of a fluorophosphoric acid on titania.

Heretofore, polyethylenepolyamine compounds such as diethylenetriamine, triethylenetetramine and the higher homologs have been produced by the reaction of an alkyl halide such as ethylene dichloride with an amine such as ammonia or ethylenediamine at elevated temperatures and pressures. Normally, relatively high yields of predominantly non-cyclic polyethylenepolyamine compounds are obtained from this process with varying yields of heterocyclic amines. The large amounts of energy required to produce the reactants as well as the difficult separation procedures required to recover the more valuable linear polyethylenepolyamines diminish the usefulness of the ethylene dichloride process. The hydrohalide salts of ammonia and the polyethylenepolyamine products must also undergo difficult and time consuming caustic neutralization to yield the free polyethylenepolyamines.

It has heretofore been known that phosphates can be used to catalyze reactions to produce predominantly heterocyclic rather than linear products. Thus, U. S. Patent No. 3,297,701 teaches the use of aluminum phosphate to catalyze the reaction of ethanolamines and polyethylenepolyamines to yield cyclic compounds. U. S. Patent No. 3,342,820 discloses the use of aluminum phosphate for the preparation of heterocyclic compounds such as triethylenediamine. As another example, U. S. Patent No. 4,103,087 also discloses the use of aluminum phosphate catalysts for producing heterocyclic product compounds.

More recently, investigators have found that more linear products can also be obtained in a catalyst conversion. Johnson et al. U. S. Patent No. 4,463,193 and U. S. Patent No. 4,578,517 are directed to the reaction of an alkanolamine with an alkyleneamine and ammonia in the presence of a catalytically effective amount of a group IIIB metal acid phosphate to give primarily noncyclic polyalkylene polyamine products. Thus, in Table 4 of U. S. Patent No. 4,463,193, Johnson et al. disclose the reaction of monoethanolamine with ethylenediamine and ammonia using catalysts such as lanthanum acid phosphate and praseodynium acid phosphate at conversions of about 11 to 43% of monoethanolamine to give a noncyclic selectivity of about 67% to 92%. In Ford et al. U. S. Patent No. 4,503,253, phosphoric acid incorporated onto an inert support (silica) was used as a catalyst and in Table 1 of the patent, use of thie type of catalyst was shown to provide monoethanolamine conversions of 34% to 68% with a selectivity to noncyclics of 62% to 86%.

European patent application 0,073,520 dated August 31, 1982 for Ford and Johnson disclosed that the reaction of monoethanolamine with ethylenediamine and ammonia can be catalyzed with acidic metal phosphates, phosphoric or phosphorous acid or their anhydrides and alkyl or aryl esters (e.g., boron phosphate, ferric phosphate, aluminum phosphate, etc.). U. S. Patent No. 4,314,083 discloses the reaction of ethylenediamine with monoethanolamine to prepare noncyclic polyalkylenepolyamines using, as a catalyst, a salt of a nitrogen or sulfur-containing compound.

In inventions originating in our laboratories, Brennan et al. in U. S. Patent No. 4,036,881 discloses the use of phosphorus-containing catalysts to catalyze the reaction of ethylenediamine with monoethanolamine. Excellent results were obtained when the reaction was conducted in an autoclave. However, when the phosphorus compound was supported on silica or diatomaceous earth, good results were obtained only at comparatively low conversions. Brennan et al. U. S. Patent No. 4,044,053 is also relevant in this regard. Brennan U. S. Patent No. 4,448,997 is directed to an alumina phosphate-type catalyst composition wherein the novel feature is the method of preparing a catalyst from alumina phosphoric acid, ammonium hydroxide and water. Excellent results were obtained using a catalyst of this nature in batch-type reactions.

More recently, Vanderpool and co-workers in a series of U. S. patents (U. S. Patent No. 4,540,822 issued September 10, 1985; U. S. Patents No. 4,578,518 and No. 4,578,519 issued March 23, 1986; U. S. Patent No. 4,584,406 issued April 22, 1986 and U. S. Patent No. 4,588,842 issued May 13, 1986) have disclosed that the reaction of monoethanolamine with ethylenediamine to provide essentially noncyclic polyethylenepolyamine reaction products can be effectively promoted with catalysts composed of a minor amount of phosphorus thermally, chemically bonded to a group IVb metal oxide support such as titania or zirconia. Also, in U. S. Patent No. 4,555,582 issued November 26, 1985, Vanderpool used a zirconium silicate catalyst to promote this reaction. Zimmerschied et. al. U. S. Patent No. 2,921,081 discloses catalysts for use in the conversion of olefins that are prepared by reacting a zirconium halide with a designated class of phosphoric acids.

Copending Knifton et al. U. S. Patent No. 4,683,335 entitled "Catalytic Process for the Preparation of Linear Polyethylenepolyamines with Tungstophosphoric Acid- or Molybdophosphoric Acid-on-Titania Catalysts" (D#80,593) discloses the process described in the title.

The use of titania-supported phosphamine catalysts for the preparation of linear polyethylenepolyamines is disclosed in my copending application S.N. 07/139,556 filed December 30, 1987 (≙ US-A-4 895 820, Publ.

23.01.90).

Rylander et. al. U. S. Patent No.2, 824,073 is concerned with the manufacture of a titanium-phosphoric acid catalyst that can be prepared by mixing titania with triphosphoric acid to form a doughy mixture which is thereafter dried and heated.

The text, "Refractories", by F. H. Norton (McGraw-Hill Book Company, Inc., 1949) in pages 318 and 319 discloses hafnium oxide, titanium oxide and zirconium oxides as well-known refractories.

Novel catalysts are disclosed which are prepared by depositing a minor amount of a fluorophosphoric acid on titania and which can be used effectively to catalyze the reaction of monoethanolamine with ethylenediamine.

Thus, the catalysts are useful in the improved production of predominantly linear polyethylenepolyamines from ethylenediamine and monoethanolamine. The novel catalysts of the claimed invention can be prepared by treating titania pellets in a manner to be described with a fluorophosphoric acid.

In one aspect of the invention the catalysts of the present invention are used in producing essentially linear polyethylenepolyamines such as diethylenetriamine, triethylenetetramine, tetraethylenepentamine and pentaethylenehexamine from the reaction of ethylenediamine and monoethanolamine.

In another aspect, the present invention is directed to an improved catalyst composition composed of titania having deposited thereon a minor amount of a fluorophosphoric acid.

The novel catalyst compositions catalyze the reaction of ethylenediamine with monoethanolamine at a temperature of from 250°C to 350°C, preferably from 270°C to 320°C and a pressure of from 500 to 3000 psig. (3.5 to 21 MPa) and preferably from 1000 to 2000 psig. (7 to 14 MPa).

Higher temperatures and pressures can be used, if desired, but there is no particular advantage in using such higher temperatures and/or pressures. The ratio of ethylenediamine to monoethanolamine may range from one to about five moles of ethylenediamine per mole of monoethanolamine.

The pelleted catalyst compositions of the present invention are preferably employed as a fixed bed of catalyst in a continuous reaction system. In a continuous process of this nature, the time of contact of the reactants with the catalyst is one of the interrelated factors that those skilled in the art will adjust, along with temperature, pressure, bed geometry, pellet size, etc. in order to obtain a desired rate of reaction and, hence, a desired percentage of conversion of the reactants. Thus, in a continuous process, it is not necessary to drive the reaction to completion because unreacted feedstock components can be recycled to the reactor.

It is customary to use cylindrically-shaped catalyst pellets having a diameter essentially equal to the length thereof, such as diameters and lengths ranging from about 0.794 mm (1/32 inch) to about 9.525 mm (3/8 inch). It will be understood that the shape and dimensions of the pellets are not critical to the present invention and that pellets of any suitable shape and dimensions may be used as desired, by one wishing to practice the process of the present invention.

When cylindrical pellets of catalyst of the type described above are used, the weighted hourly space velocity may be varied within wide limits (e.g., 0.1 to 5 w/hr/w) in order to obtain a desired rate of conversion, as explained above. Normally, space velocities of about 0.5 to 2 w/hr/w will be employed.

Catalyst life is an important factor in conducting a continuous reaction. For example, if a catalyst is easily poisoned, or if catalyst pellets do not have good structural properties, the economics of the process will be seriously and adversely affected.

The catalysts of the present invention are not particularly susceptible to poisoning so this normally does not present a problem. However, under the reaction conditions employed, amines of the type used and formed herein have the potential capability of leaching or otherwise adversely affecting the structural integrity of the pellets. In an extreme instance, catalyst pellets having good initial crush strength and surface hardness will be reduced to fines very rapidly when used under reaction conditions such as those employed herein.

As a consequence, the catalyst compositions of the present invention are advantageously used for a continuous process for the continuous production of essentially linear polyethylenepolyamine reaction products from monoethanolamine and ethylenediamine. Such catalyst compositions can be used for prolonged periods without the need for regeneration (e.g., 1,000 hours or more). Nevertheless, with the passage of time deactivation will tend to slowly occur. Deactivation can be measured qualitatively as the increase of temperature required to maintain an essentially constant conversion rate for the monoethanolamine and ethylenediamine.

The catalyst compositions of the present invention are prepared by depositing a minor amount of a fluorophosphoric acid on titania. Fluorophosphoric acid and difluorophosphoric acid have been used with good results. Titania pellets can be prepared by extrusion or by compaction in conventional pelleting apparatus using a pelleting aid such as graphite.

As indicated, the solvent for the fluorophosphoric acid to be used for the impregnation of the titania pellets may be water or any of the known polar organic solvents such as alcohols, ketones, etc. Examples of suitable polar organic solvents that may be used include compounds such as methanol, ethanol, n-propanol, isopropyl

alcohol, n-butyl alcohol, t-butyl alcohol, acetone, methyl ethyl ketone, etc.

The catalyst composition is prepared by impregnating preformed pellets of titania. A suitable procedure to be used is to immerse the titania pellets in an aqueous solution or a polar organic solvent solution of the fluorophosphoric acid, preferably at ambient temperatures (about 20° to about 30°C). Higher temperatures of up to about 110°C can be used, if desired. Thus, the impregnation temperature to be used will normally be within the range of about 20° to about 110°C, preferably within the range of 20° to 55°C.

The solution of the fluorophosphoric acid that is used for the impregnation step is suitably about a 10% to about a 50% solution of the fluorophosphoric acid in water or a polar organic solvent. For example, the solution may contain from about 15% to about 25% of the fluorophosphoric acid.

The immersion treatment is continued, preferably with agitation, for a period of about 0.1 to about 5 hours sufficient to permit the solution to penetrate the pores of the titania pellets. Suitably, the amount of solution of the fluorophosphoric acid that is used should be adequate to permit full immersion of the titania pellets. Larger amounts of the solution can be used, if desired, but there is no particular advantage in doing so. At the end of the immersion step, the excess solution can be evaporated from the treated pellets or the pellets can be removed from the solution and permitted to dry (e.g., in a drying oven).

Only a minor amount of the fluorophosphoric acid will be permanently deposited on the titania pellets by this procedure, such that the treated titania pellets will have only about 0.01 to about 5 wt.% of phosphorus deposited thereon, and normally about 2 wt.% or less (e.g., 0.1 to 2 wt.%).

It will be understood that the phosphorus that is present on thus-treated titania pellets is not present as an elemental compound, but rather as fluorophosphorus groups that are chemically bound, normally as an oxide, to the titania support. The exact nature of the bonding is not completely understood, but is believed to be as follows:

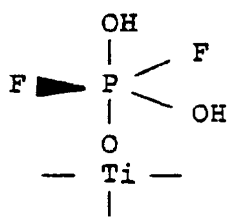

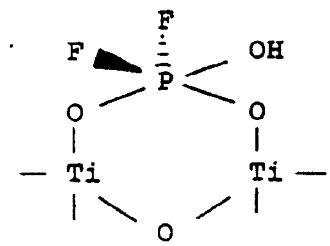

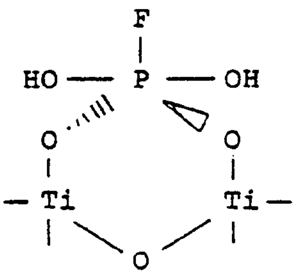

The pelleted catalyst compositions of the present invention should be calcined. They can be calcined prior to use or calcined in situ when used as catalysts at temperatures in excess of about 100°C. When the catalysts are to be calcined prior to use, calcination is suitably conducted for 2 to 24 hours at a temperature above about 100°C but below the temperature at which thermal destruction of the chemical bonding occurs. This can be determined by routine experimentation for a particular catalyst. Temperatures above 650°C should be avoided. A suitable calcining temperature range is normally 100° to 650°C, preferably 200° to 400°C and still more preferably 300° to 350°C.

There are many compounds which can be formed from the reaction of ethylenediamine and monoethanolamine besides the preferred linear polyethylenepolyamines such as diethylenetriamine, triethylenetetramine, tetraethylenepentamine and pentaethylenehexamine. Less desirable cyclics and other compounds, such as piperazine, N-(2-aminoethyl)ethanolamine and N-(2-aminoethyl)piperazine, are also formed. The more desired linear polyethylenepolyamines can be easily recovered from the reaction product mixture by conventional methods such as distillation. Such distillation recovery methods are well known in the art. An outstanding advantage of the claimed invention is that the lower molecular weight polyethylenepolyamines recovered from the reaction mixture can be further reacted with monoethanolamine to produce a larger percentage of the higher molecular weight linear polyethylenepolyamines.

Ammonia may also be added as a reactant in the generation of the preferred linear polyethylenepolyamines from ethylenediamine and monoethanolamine using the fluorophosphoric acid on titania catalysts of this invention.

In the drawings:

Fig. 1 is a graph wherein data have been plotted showing the effect of reactor temperature on the conversion of monoethanolamine to non-cyclic products when monoethanolamine is reacted with ethylenediamine and the reaction is catalyzed with a preferred catalyst of the present invention and, for reference purposes, data is plotted showing the same effect for a reference catalyst known, on the basis of past experience, to give outstanding results, and

Fig. 2 is a graph wherein data have been plotted showing the effect of reactor temperature on the selectivity of monoethanolamine to non-cyclic products when monoethanolamine is reacted with ethylenediamine and the reaction is catalyzed with a preferred catalyst of the present invention and, for reference purposes, data is plotted showing the same effect for a reference catalyst known, on the basis of past experience, to give outstanding results.

## SPECIFIC EXAMPLES

The following examples will further illustrate the preparation of predominantly linear polyethylenepolyamines from ethylenediamine and monoethanolamine by the use of the catalyst compositions of the present invention. They are given by way of illustration and not as limitations on the scope of the invention. Thus, it will be understood that reactants, proportions of reactants, and time, temperature and pressure of the reaction steps may be varied with much the same results achieved.

For purposes of convenience and brevity, the reactant compounds employed and the products obtained have been abbreviated in the following examples and tables. The abbreviations employed for these various compounds are:

EDA - ethylenediamine
MEA - monoethanolamine
PIP - piperazine
BAEE - bisaminoethyl ether
DETA - diethylenetriamine
TETA - triethylenetetramine
TEPA - tetraethylenepentamine
AEEA - N-(2-aminoethyl)ethanolamine
AEP - N-(2-aminoethyl)piperazine
HEP - N-(hydroxyethyl)piperazine
NTEA - nitrilo tris ethyleneamine
DIAEP - diaminoethyl piperazine
PEEDA - piperazino ethyl-ethylenediamine
AETETA - aminoethyl triethylene tetramine

The following examples of the present invention are given by way of illustration only and are not intended as limitations on the scope of the invention which is defined by the appended claims.

### 1. Catalyst Preparation (6147-21)

A preformed pelleted extrudate titanium dioxide catalyst support (178g 200cc) was placed in 11 roundbottom flask. A solution of fluorophosphoric acid (11.5g) in 80cc of reagent acetone (62g) was added. The flask was placed on a rotary evaporator and evacuated to aspirator vacuum then heated to 55°C for 1 hour with periodic stirring. The resultant mixture was placed in a glass tube 1″x2′/and heated to 150°C for 30 min. Thereafter, it was heated at 350°C under nitrogen flow for 2 hours. Both the calcined and uncalcined catalyst contained 2% P by AA.

### 2. Use as a Catalyst for Formation of Polyethylenepolyamines (6147-22)

The above catalyst was placed in a stainless steel tube in an aluminum heating block (100cc of catalyst). Through the tube was passed a mixture of EDA (2pbw) and MEA (1pbw) at a rate of 100cc per hour. The apparatus was held at a series of temperatures ranging from about 250° to about 320°C, being held at each temperature for about 3 to 4 hours to allow equilibration. Samples were taken for analysis by GLC. The area % analysis along with the calculated % MEA conversion, % EDA conversion, DETA/Piperazine ratio, and percent non-cyclic products in the TETA range as well as the relative selectivities among the observed products is presented for each of the samples in Table I. The reaction temperature at which the sample was taken is also noted.

TABLE I-A

Ethyleneamines Continuous Reactor Products

| 1<br>Sample<br>6147-22 | 2<br>Temp<br>°C | 3<br>Area<br>% EDA | 4<br>Area<br>% MEA | 5<br>% MEA<br>Conv. | 6<br>Area<br>% PIP | 7<br>% Selec.<br>to PIP | 8<br>Area<br>% BAEE | 9<br>% Selec.<br>to BAEE |
|---|---|---|---|---|---|---|---|---|
| Feed | | 65.75 | 34.23 | | | | | |
| 1 | 290 | 62.42 | 22.03 | 35.62 | 0.14 | 0.97 | 0.17 | 1.13 |
| 2 | 290 | 60.38 | 23.06 | 32.61 | 0.10 | 0.65 | 0.00 | 0.00 |
| 3 | 289 | 62.34 | 21.78 | 36.35 | 0.21 | 1.37 | 0.00 | 0.00 |
| 4 | 250 | 66.82 | 30.28 | 11.52 | 0.00 | 0.00 | 0.00 | 0.00 |
| 5 | 249 | 64.15 | 31.64 | 7.56 | 0.00 | 0.00 | 0.00 | 0.00 |
| 6 | 259 | 64.11 | 30.39 | 11.19 | 0.01 | 0.23 | 0.00 | 0.00 |
| 7 | 260 | 64.56 | 30.04 | 12.21 | 0.02 | 0.42 | 0.00 | 0.00 |
| 8 *rep | 272 | 63.87 | 27.64 | 19.25 | 0.06 | 0.76 | 0.00 | 0.00 |
| 9 | 270 | 64.73 | 28.43 | 16.94 | 0.04 | 0.66 | 0.00 | 0.00 |
| 10 | 279 | 63.22 | 25.59 | 25.21 | 0.08 | 0.77 | 0.00 | 0.00 |
| 11 | 280 | 63.13 | 25.56 | 25.32 | 0.06 | 0.57 | 0.00 | 0.00 |
| 12 | 290 | 62.71 | 21.10 | 38.34 | 0.29 | 1.86 | 0.00 | 0.00 |
| 13 | 290 | 63.32 | 21.08 | 38.40 | 0.12 | 0.83 | 0.00 | 0.00 |
| 14 | 299 | 59.64 | 17.20 | 49.75 | 0.40 | 1.79 | 0.00 | 0.00 |
| 15 | 298 | 59.22 | 18.07 | 47.20 | 0.29 | 1.30 | 0.00 | 0.00 |
| 16 | 310 | 57.64 | 11.73 | 65.72 | 0.64 | 2.18 | 0.04 | 0.14 |
| 17 | 310 | 55.73 | 12.39 | 63.78 | 0.63 | 2.07 | 0.00 | 0.00 |
| 18 | 320 | 53.37 | 6.32 | 81.52 | 0.94 | 2.49 | 0.01 | 0.04 |
| 19 | 320 | 53.29 | 6.80 | 80.11 | 0.97 | 2.59 | 0.00 | 0.00 |
| 20 | 300 | 57.84 | 17.23 | 49.63 | 0.44 | 1.83 | 0.00 | 0.00 |

TABLE I-B

| 1 Sample 6147-22 | 10 % Conv. of EDA | 11 Area % DETA | 12 % Selec. to DETA | 13 Area % AEEA | 14 % Selec. to AEEA | 15 Ratio: Obsv. DETA/ Obsv. PIP | 16 Area % AEP |
|---|---|---|---|---|---|---|---|
| 1 | 5.07 | 13.44 | 89.93 | 0.28 | 1.88 | 92.70 | 0.08 |
| 2 | 8.16 | 14.57 | 89.63 | 0.38 | 2.34 | 137.51 | 0.11 |
| 3 | 5.18 | 14.39 | 91.75 | 0.46 | 2.93 | 66.94 | 0.09 |
| 4 | -1.61 | 2.38 | 100.00 | 0.00 | 0.00 | ?? | 0.00 |
| 5 | 2.44 | 4.21 | 100.00 | 0.00 | 0.00 | ?? | 0.00 |
| 6 | 2.49 | 5.41 | 98.77 | 0.00 | 0.00 | 416.61 | 0.05 |
| 7 | 1.80 | 5.09 | 97.90 | 0.00 | 0.00 | 231.45 | 0.08 |
| 8 | 2.86 | 8.15 | 96.48 | 0.21 | 2.56 | 125.47 | 0.00 |
| 9 | 1.55 | 6.12 | 96.86 | 0.15 | 2.46 | 145.78 | 0.00 |
| 10 | 3.84 | 10.54 | 95.30 | 0.39 | 3.53 | 122.66 | 0.04 |
| 11 | 3.99 | 10.68 | 95.28 | 0.39 | 3.47 | 164.43 | 0.03 |
| 12 | 4.62 | 14.86 | 93.21 | 0.53 | 3.32 | 49.88 | 0.13 |
| 13 | 3.70 | 14.61 | 94.77 | 0.44 | 2.91 | 114.17 | 0.11 |
| 14 | 9.29 | 18.29 | 80.24 | 0.52 | 2.30 | 44.73 | 0.26 |
| 15 | 9.93 | 18.27 | 81.51 | 0.48 | 2.18 | 62.57 | 0.26 |
| 16 | 12.33 | 21.53 | 73.32 | 0.29 | 0.99 | 33.59 | 0.52 |
| 17 | 15.23 | 22.58 | 73.86 | 0.26 | 0.86 | 35.55 | 0.55 |
| 18 | 18.82 | 25.88 | 68.08 | 0.08 | 0.23 | 27.30 | 1.14 |
| 19 | 18.95 | 25.25 | 67.20 | 0.12 | 0.32 | 25.87 | 1.11 |
| 20 | 12.03 | 19.17 | 79.39 | 0.40 | 1.69 | 43.19 | 0.33 |

TABLE I-C

| 1 Sample 6147-22 | 17 % Selec. of AEP | 18 Area % NTEA | 19 % Selec. to NTEA | 20 % NC | 21 Area % TETA | 22 % Selec. of TETA | 23 Area % DIAEP |
|---|---|---|---|---|---|---|---|
| 1 | 0.59 | 0.07 | 0.49 | 100.00 | 0.74 | 4.98 | 0.00 |
| 2 | 0.68 | 0.09 | 0.57 | 100.00 | 0.99 | 6.09 | 0.00 |
| 3 | 0.63 | 0.10 | 0.69 | 100.00 | 0.41 | 2.61 | 0.00 |
| 4 | 0.00 | 0.00 | 0.00 | ?? | 0.00 | 0.00 | 0.00 |
| 5 | 0.00 | 0.00 | 0.00 | ?? | 0.00 | 0.00 | 0.00 |
| 6 | 0.98 | 0.00 | 0.00 | ?? | 0.00 | 0.00 | 0.00 |
| 7 | 1.67 | 0.00 | 0.00 | ?? | 0.00 | 0.00 | 0.00 |
| 8 | 0.00 | 0.00 | 0.00 | 0.00 | 0.00 | 0.00 | 0.00 |
| 9 | 0.00 | 0.00 | 0.00 | ?? | 0.00 | 0.00 | 0.00 |
| 10 | 0.38 | 0.00 | 0.00 | ?? | 0.00 | 0.00 | 0.00 |
| 11 | 0.33 | 0.00 | 0.00 | 100.00 | 0.03 | 0.32 | 0.00 |
| 12 | 0.85 | 0.11 | 0.73 | 100.00 | 0.00 | 0.00 | 0.00 |
| 13 | 0.71 | 0.11 | 0.77 | 100.00 | 0.00 | 0.00 | 0.00 |
| 14 | 1.14 | 0.29 | 1.27 | 98.85 | 2.89 | 12.69 | 0.00 |
| 15 | 1.16 | 0.26 | 1.19 | 98.76 | 2.75 | 12.30 | 0.00 |
| 16 | 1.77 | 0.47 | 1.61 | 97.46 | 4.82 | 16.42 | 0.06 |
| 17 | 1.82 | 0.48 | 1.58 | 97.42 | 4.95 | 16.21 | 0.06 |
| 18 | 3.01 | 0.67 | 1.76 | 96.25 | 7.35 | 19.34 | 0.16 |
| 19 | 2.95 | 0.67 | 1.80 | 95.68 | 7.26 | 19.33 | 0.16 |
| 20 | 1.38 | 0.32 | 1.34 | 98.65 | 3.27 | 13.56 | 0.00 |

TABLE I-D

| 1 Sample 6147-22 | 24 % Selec. DIAEP | 25 Area % PEEDA | 26 % Selec. PEEDA | 27 Area % AETETA | 28 % Selec. AETETA | 29 Area % TEPA | 30 % Selec. TEPA | 31 Total Product |
|---|---|---|---|---|---|---|---|---|
| 1 | 0.00 | 0.00 | 0.00 | 0.00 | 0.00 | 0.00 | 0.00 | 14.94 |
| 2 | 0.00 | 0.00 | 0.00 | 0.00 | 0.00 | 0.00 | 0.00 | 16.26 |
| 3 | 0.00 | 0.00 | 0.00 | 0.00 | 0.00 | 0.00 | 0.00 | 15.68 |
| 4 | 0.00 | 0.00 | 0.00 | 0.00 | 0.00 | 0.00 | 0.00 | 2.38 |
| 5 | 0.00 | 0.00 | 0.00 | 0.00 | 0.00 | 0.00 | 0.00 | 4.21 |
| 6 | 0.00 | 0.00 | 0.00 | 0.00 | 0.00 | 0.00 | 0.00 | 5.48 |
| 7 | 0.00 | 0.00 | 0.00 | 0.00 | 0.00 | 0.00 | 0.00 | 5.20 |
| 8 | 0.00 | 0.01 | 0.17 | 0.00 | 0.00 | 0.00 | 0.00 | 8.45 |
| 9 | 0.00 | 0.00 | 0.00 | 0.00 | 0.00 | 0.00 | 0.00 | 6.32 |
| 10 | 0.00 | 0.00 | 0.00 | 0.00 | 0.00 | 0.00 | 0.00 | 11.06 |
| 11 | 0.00 | 0.00 | 0.00 | 0.00 | 0.00 | 0.00 | 0.00 | 11.21 |
| 12 | 0.00 | 0.00 | 0.00 | 0.00 | 0.00 | 0.00 | 0.00 | 15.94 |
| 13 | 0.00 | 0.00 | 0.00 | 0.00 | 0.00 | 0.00 | 0.00 | 15.42 |
| 14 | 0.00 | 0.03 | 0.16 | 0.00 | 0.00 | 0.08 | 0.38 | 22.79 |
| 15 | 0.00 | 0.03 | 0.16 | 0.00 | 0.00 | 0.03 | 0.16 | 22.41 |
| 16 | 0.21 | 0.07 | 0.25 | 0.36 | 1.24 | 0.53 | 1.82 | 29.36 |
| 17 | 0.20 | 0.08 | 0.26 | 0.40 | 1.32 | 0.54 | 1.77 | 30.56 |
| 18 | 0.43 | 0.14 | 0.38 | 0.58 | 1.52 | 1.01 | 2.66 | 38.02 |
| 19 | 0.42 | 0.19 | 0.52 | 0.62 | 1.65 | 1.19 | 3.17 | 37.57 |
| 20 | 0.00 | 0.04 | 0.20 | 0.00 | 0.00 | 0.14 | 0.57 | 24.15 |

3. Data for Comparative Graphs

In order to evaluate the relative effectiveness of the fluorophosphoric acid-on-titania catalyst of the present invention, comparative data was obtained using a phosphoric acid-on-titania catalyst that had been used extensively, and with outstanding results, the reference catalyst having been prepared by depositing about 5% of phosphoric acid on titania. The comparative data was obtained by reacting monoethanolamine with ethylenediamine in order to provide reaction products consisting essentially of linear polyethylenepolyamines, the equipment and procedure of Example 2 being used for this purpose. Tests were run at a plurality of temperatures ranging from about 300° to about 326°C and the reaction products were sampled and analyzed in order

to obtain data from which the % of monoethanolamine conversion and the yield of non-cyclic products was calculated.

The calculated data obtained from the comparative tests and representative data obtained with catalyst 6147-21 of the present invention were used to prepare the graphs of Figs. 1 and 2. The data used in preparing the graphs of Figs. 1 and 2 are also tabularly recorded, as set forth herein in the following Table II and Table III.

TABLE II

Data for Comparative Graphs

Conversion vs. Temperature (°C)

| Commercial Phosphate on Titania | | 6147-21 Fluorophosphate on Titania | |
|---|---|---|---|
| Temp. | % MEA Conv. | Temp. | % MEA Conv. |
| 300 | 38.3 | 249 | 7.56 |
| 305 | 45.9 | 250 | 11.52 |
| 311 | 59.4 | 259 | 11.19 |
| 314 | 64.5 | 260 | 12.21 |
| 320 | 65.9 | 270 | 16.94 |
| 322 | 73.2 | 272 | 19.25 |
| 326 | 99.9 | 279 | 25.21 |
| | | 280 | 25.32 |
| | | 289 | 36.35 |
| | | 290 | 35.62 |
| | | 290 | 35.61 |
| | | 290 | 38.34 |
| | | 298 | 47.20 |
| | | 299 | 49.75 |
| | | 300 | 49.63 |
| | | 310 | 65.72 |
| | | 310 | 63.78 |
| | | 320 | 81.52 |
| | | 320 | 80.11 |

## TABLE III

## % Non-Cyclics in the TETA Range

| Commercial Phosphate on Titania Temp. (°C) | Commercial Phosphate on Titania % Non-Cyclics | 6147-21 Fluorophosphate on Titania Temp. (°C) | 6147-21 Fluorophosphate on Titania % Non-Cyclics |
|---|---|---|---|
| 300 | 87.8 | 249 | * |
| 305 | 80.7 | 250 | * |
| 311 | 79.4 | 259 | * |
| 314 | 77.8 | 260 | * |
| 320 | 75.4 | 270 | * |
| 322 | 77.0 | 272 | * |
| 326 | 73.2 | 279 | * |
| | | 280 | 100** |
| | | 289 | 100** |
| | | 290 | 100** |
| | | 290 | 100** |
| | | 290 | 100** |
| | | 298 | 98.76 |
| | | 299 | 98.85 |
| | | 300 | 98.65 |
| | | 310 | 97.46 |
| | | 310 | 97.42 |
| | | 320 | 96.25 |
| | | 320 | 95.68 |

* peaks >0.01% were not observed in the TETA range
** no peaks >0.01% observed for non-cyclic in TETA range

Turning now to Fig. 1, it will be observed that the catalyst of the present invention was clearly superior, giving about a 10% improvement in MEA conversion, as compared with the reference catalyst.

The results are even more dramatic when the data on selectivity to non-cyclic products in the TETA range for the catalyst of the present invention is compared with the data for the reference catalyst. Thus, the selectivity is significantly higher at 300°C (about 98%) using the catalyst of the present invention, as compared with a selectivity of only about 86% using the reference catalyst.

What is even more significant, however, is that selectivity was about 95% at 320°C (a drop of only about 3%) whereas selectivity for the reference catalyst had dropped to a clearly unsatisfactory level of about 75% (a drop of about 9%).

4. Continuous One Thousand Hour Test Run Using Catalyst 6147-21 while Reacting Monoethanolamine with Ethylenediamine

A mixture of monoethanolamine (1 pbw) with ethylenediamine (2 pbw) was passed through the apparatus of Example 2 at the rate of about 100 cc per hour for about one thousand hours at a temperature of about 305° to about 310°C. The reaction product was periodically sampled and analyzed. The conversion of monoethanolamine remained essentially constant at about 65% during the test period and the combined selectivity to diethylenetriamine and triethylenetetramine was also essentially constant, varying between about 92% and 93%.

The results are reported in greater detail in Table IV.

TABLE IV-A

Ethyleneamines Continuous Reactor Products Using 6147-21 Fluorophosphate on Titania Catalyst

| 1<br>Sample 6147-23 | 2<br>Temp °C | 3<br>Time (hr) | 4<br>Area % EDA | 5<br>Area % MEA | 6<br>% MEA Conv. | 7<br>Area % PIP | 8<br>% Selec. to PIP | 9<br>Area % BAEE |
|---|---|---|---|---|---|---|---|---|
| Feed | | | 67.23 | 32.77 | | | | |
| 1 | 305 | 9 | 57.12 | 15.80 | 51.77 | 0.41 | 1.54 | 0.00 |
| 2 | 305 | 16 | 60.28 | 14.88 | 54.60 | 0.51 | 2.09 | 0.00 |
| 3 | 305 | 65 | 56.84 | 15.52 | 52.64 | 0.52 | 1.93 | 0.00 |
| 4 | 305 | 110 | 58.92 | 14.92 | 54.46 | 0.44 | 1.74 | 0.00 |
| 5 | 307 | 164 | 60.87 | 13.98 | 57.36 | 0.39 | 1.63 | 0.00 |
| 6 | 306 | 183 | 62.03 | 13.63 | 58.42 | 0.49 | 2.07 | 0.00 |
| 7 | 307 | 255 | 60.59 | 13.50 | 58.81 | 0.45 | 1.83 | 0.00 |
| 8 | 309 | 303 | 63.11 | 11.96 | 63.50 | 0.46 | 1.93 | 0.00 |
| 9 | 307 | 347 | 62.03 | 13.36 | 59.23 | 0.52 | 2.23 | 0.05 |
| 10 | 308 | 354 | 61.76 | 12.67 | 61.33 | 0.55 | 2.24 | 0.00 |
| 11 | 307 | 426 | 61.34 | 14.44 | 55.93 | 0.44 | 1.89 | 0.01 |
| 12 | 307 | 443 | 62.78 | 13.27 | 59.51 | 0.53 | 2.31 | 0.04 |
| 18 | 309 | 690 | 62.99 | 13.56 | 58.63 | 0.58 | 2.56 | 0.00 |
| 19 | 312 | 792 | 62.94 | 12.79 | 60.97 | 0.49 | 2.07 | 0.00 |
| 20 | 307 | 864 | 61.54 | 16.84 | 48.61 | 1.28 | 6.01 | 0.00 |
| 21 | 308 | 960 | 63.38 | 14.67 | 55.23 | 0.48 | 2.28 | 0.00 |
| 22 | 306 | 1032 | 62.28 | 17.87 | 45.49 | 1.35 | 6.84 | 0.00 |
| 23 | 306 | 1128 | 62.05 | 17.81 | 45.66 | 0.42 | 2.07 | 0.00 |

TABLE IV-B

| 1 Sample 6147-23 | 10 % Selec. BAEE | 11 % Conv. of EDA | 12 Area % DETA | 13 % Selec. DETA | 14 Area % AEEA | 15 % Selec. to AEEA | 16 Ratio: Obsv. DETA/ Obsv. PIP | 17 Area % AEP |
|---|---|---|---|---|---|---|---|---|
| 1 | 0.00 | 15.04 | 20.51 | 77.51 | 0.36 | 1.34 | 50.26 | 0.44 |
| 2 | 0.00 | 10.34 | 19.01 | 78.57 | 0.38 | 1.58 | 37.64 | 0.36 |
| 3 | 0.00 | 15.45 | 20.41 | 76.65 | 0.32 | 1.20 | 39.63 | 0.45 |
| 4 | 0.00 | 12.36 | 20.16 | 79.86 | 0.23 | 0.92 | 45.81 | 0.46 |
| 5 | 0.00 | 9.46 | 19.41 | 80.57 | 0.37 | 1.54 | 49.51 | 0.40 |
| 6 | 0.00 | 7.74 | 18.37 | 78.36 | 0.37 | 1.56 | 37.87 | 0.34 |
| 7 | 0.00 | 9.88 | 19.38 | 78.57 | 0.34 | 1.39 | 42.98 | 0.42 |
| 8 | 0.00 | 6.12 | 20.10 | 83.61 | 0.13 | 0.54 | 43.33 | 0.54 |
| 9 | 0.20 | 7.73 | 18.54 | 79.72 | 0.22 | 0.95 | 35.80 | 0.43 |
| 10 | 0.00 | 8.14 | 19.29 | 78.75 | 0.25 | 1.02 | 35.13 | 0.48 |
| 11 | 0.05 | 8.76 | 18.29 | 79.38 | 0.29 | 1.25 | 41.96 | 0.47 |
| 12 | 0.17 | 6.62 | 17.78 | 77.80 | 0.28 | 1.22 | 33.73 | 0.41 |
| 18 | 0.00 | 6.31 | 17.84 | 79.28 | 0.21 | 0.93 | 30.97 | 0.35 |
| 19 | 0.00 | 6.39 | 18.52 | 78.11 | 0.25 | 1.05 | 37.79 | 0.41 |
| 20 | 0.00 | 8.47 | 16.09 | 75.41 | 0.34 | 1.61 | 12.55 | 0.29 |
| 21 | 0.00 | 5.72 | 16.39 | 78.45 | 0.35 | 1.68 | 34.44 | 0.34 |
| 22 | 0.00 | 7.36 | 14.91 | 75.33 | 0.37 | 1.88 | 11.02 | 0.27 |
| 23 | 0.00 | 7.71 | 15.73 | 78.43 | 0.39 | 1.93 | 37.91 | 0.29 |

TABLE IV-C

| 1 Sample 6147-23 | 18 % Selec. of AEP | 19 Area % NTEA | 20 % Selec. to NTEA | 21 % NC | 22 Area % TETA | 23 % Selec. of TETA | 24 Area % DIAEP |
|---|---|---|---|---|---|---|---|
| 1 | 1.64 | 0.42 | 1.58 | 95.35 | 3.96 | 14.95 | 0.00 |
| 2 | 1.47 | 0.31 | 1.29 | 98.62 | 3.33 | 13.78 | 0.00 |
| 3 | 1.70 | 0.37 | 1.37 | 98.35 | 3.94 | 14.78 | 0.00 |
| 4 | 1.83 | 0.34 | 1.36 | 94.68 | 3.40 | 13.45 | 0.00 |
| 5 | 1.65 | 0.30 | 1.24 | 98.28 | 3.02 | 12.53 | 0.00 |
| 6 | 1.45 | 0.30 | 1.26 | 98.96 | 3.41 | 14.54 | 0.00 |
| 7 | 1.69 | 0.31 | 1.24 | 98.61 | 3.54 | 14.33 | 0.00 |
| 8 | 2.25 | 0.21 | 0.86 | 100.00 | 2.60 | 10.81 | 0.00 |
| 9 | 1.85 | 0.26 | 1.13 | 100.00 | 3.10 | 13.34 | 0.00 |
| 10 | 1.95 | 0.31 | 1.25 | 100.00 | 3.43 | 14.02 | 0.00 |
| 11 | 2.03 | 0.27 | 1.16 | 98.46 | 3.07 | 13.31 | 0.00 |
| 12 | 1.78 | 0.28 | 1.21 | 98.27 | 3.18 | 13.91 | 0.00 |
| 18 | 1.57 | 0.00 | 0.00 | 100.00 | 3.48 | 15.45 | 0.00 |
| 19 | 1.73 | 0.00 | 0.00 | 100.00 | 3.99 | 16.83 | 0.00 |
| 20 | 1.37 | 0.00 | 0.00 | 100.00 | 3.23 | 15.15 | 0.00 |
| 21 | 1.61 | 0.00 | 0.00 | 100.00 | 3.24 | 15.50 | 0.00 |
| 22 | 1.34 | 0.00 | 0.00 | 100.00 | 2.80 | 14.12 | 0.00 |
| 23 | 1.43 | 0.00 | 0.00 | 100.00 | 3.03 | 15.10 | 0.00 |

TABLE IV-D

| 1 Sample 6147-23 | 25 % Selec. DIAEP | 26 Area % PEEDA | 27 % Selec. PEEDA | 28 Area % AETETA | 29 % Selec. AETETA | 30 Area % TEPA | 31 % Selec. TEPA | 32 Total Product |
|---|---|---|---|---|---|---|---|---|
| 1 | 0.00 | 0.21 | 0.81 | 0.02 | 0.06 | 0.15 | 0.57 | 26.46 |
| 2 | 0.00 | 0.05 | 0.21 | 0.16 | 0.66 | 0.09 | 0.35 | 24.19 |
| 3 | 0.00 | 0.07 | 0.27 | 0.27 | 1.03 | 0.29 | 1.07 | 26.63 |
| 4 | 0.00 | 0.21 | 0.83 | 0.00 | 0.00 | 0.00 | 0.00 | 25.24 |
| 5 | 0.00 | 0.06 | 0.24 | 0.01 | 0.05 | 0.13 | 0.55 | 24.09 |
| 6 | 0.00 | 0.04 | 0.17 | 0.01 | 0.06 | 0.13 | 0.53 | 23.44 |
| 7 | 0.00 | 0.05 | 0.22 | 0.02 | 0.06 | 0.16 | 0.66 | 24.67 |
| 8 | 0.00 | 0.00 | 0.00 | 0.00 | 0.00 | 0.00 | 0.00 | 24.04 |
| 9 | 0.00 | 0.00 | 0.00 | 0.02 | 0.06 | 0.12 | 0.52 | 23.26 |
| 10 | 0.00 | 0.00 | 0.00 | 0.02 | 0.08 | 0.17 | 0.68 | 24.49 |
| 11 | 0.00 | 0.05 | 0.23 | 0.02 | 0.09 | 0.14 | 0.62 | 23.05 |
| 12 | 0.00 | 0.06 | 0.27 | 0.18 | 0.78 | 0.13 | 0.56 | 22.85 |
| 18 | 0.00 | 0.00 | 0.00 | 0.05 | 0.21 | 0.00 | 0.00 | 22.50 |
| 19 | 0.00 | 0.00 | 0.00 | 0.05 | 0.22 | 0.00 | 0.00 | 23.71 |
| 20 | 0.00 | 0.00 | 0.00 | 0.03 | 0.14 | 0.07 | 0.30 | 21.34 |
| 21 | 0.00 | 0.00 | 0.00 | 0.03 | 0.13 | 0.08 | 0.36 | 20.89 |
| 22 | 0.00 | 0.00 | 0.00 | 0.02 | 0.12 | 0.07 | 0.36 | 19.79 |
| 23 | 0.00 | 0.00 | 0.00 | 0.02 | 0.11 | 0.19 | 0.93 | 20.06 |

With reference to Table IV, it is to be noted that the conversion of monoethanolamine remained high during the entire 1,000 hour period. Thus, conversion was 51.77% at 305°C after 9 hours and was 55.23% at 308°C after 960 hours; conversion dropping below 50% only once during the 1,000 run for Sample #20 at 307°C after 864 hours. Conversion did drop below 50% thereafter for samples 22 and 23 at 1,032 hours and 1,128 hours, respectively.

However, and even more significantly, the selectivity of the reaction to non-cyclic products remained uniformly high during the entire 1,128 hours, especially insofar as the selectivity to triethylenetetramine was concerned.

5. Catalyst Preparation - Catalyst 6147-47 Difluorophosphoric Acid on Titania

A catalyst was prepared from a preformed pelleted titanium dioxide catalyst support and difluorophosphoric

acid. About 220 cc (about 240g) of the titanium dioxide pellets were placed in a polypropylene mixing bowl and a solution of about 17.2g of difluorophosphoric acid in about 125 cc (about 95.98g) of acetone was added. After stirring, the mixing bowl was placed in a vacuum dessicator and evacuated at aspirator vacuum until dry. The thus-impregnated titania pellets were then transferred to a 1 1. round bottomed flask and heated at one hour at about 80°C on a rotary evaporator. Thereafter, the catalyst was heated at 350°C under nitrogen flow for about 2 hours. A sample of the catalyst analyzed by atomic absorption spectrometry gave an analysis result of about 0.64 wt.% of phosphorus. Analysis by ion chromotography gave an analysis result of about 0.52 wt.% of phosphorus.

6. Use of Difluorophosphoric Acid-on-Titania to Catalyze the Reaction of Monoethanolamine with Ethylenediamine

About 100 cc of the difluorophosphoric acid-on-titania catalyst prepared in Example 5 (catalyst 6147-47) was placed in a stainless steel tube in an aluminum block and a mixture of monoethanolamine (1 pbw) with ethylenediamine (2 pbw) was passed through the tube at the rate of about 100 cc per hour. Several runs were made at reaction temperatures of from about 290° to about 320°C. For each run, the apparatus was held at the desired temperature for about 3 to 4 hours to allow equilibration and then the reaction product was sampled. The samples were analyzed by GLC. Data on the results of the runs are given in Table V where each of the samples is identified, together with the temperature at which it was run. The area % analysis along with the calculated % MEA conversion, the % DEA conversion, the DETA/piperazine ratio and percent of non-cyclic products in the TETA range as well as the relative selectivities among the observed products is presented for each of the samples in Table V.

TABLE V-A

Ethyleneamines Continuous Reactor Products
Difluorophosphoric Acid on Titania (6147-47)

| 1 Sample 6147-59 | 2 Temp. °C | 3 Area % EDA | 4 Area % MEA | 5 % MEA Conv. | 6 Area % PIP | 7 % Selec. to PIP | 8 Area % BAEE | 9 % Selec. to BAEE | 10 % Conv. of EDA | 11 Area % DETA | 12 % Selec. to DETA |
|---|---|---|---|---|---|---|---|---|---|---|---|
| 1 | 301 | 55.84 | 15.92 | 53.17 | 0.55 | 2.01 | 0.06 | 0.22 | 15.40 | 20.44 | 74.11 |
| 2 | 303 | 54.22 | 13.96 | 58.95 | 0.73 | 2.37 | 0.05 | 0.17 | 17.85 | 22.36 | 72.19 |
| 3 | 280 | 62.52 | 24.06 | 29.24 | 0.22 | 1.63 | 0.02 | 0.18 | 5.27 | 11.12 | 83.71 |
| 4 | 292 | 58.09 | 19.51 | 42.62 | 0.44 | 2.01 | 0.04 | 0.19 | 11.98 | 16.16 | 74.35 |
| 5 | 299 | 56.97 | 17.16 | 49.52 | 0.62 | 2.45 | 0.07 | 0.27 | 13.68 | 19.41 | 76.65 |
| 6 | 310 | 52.49 | 11.88 | 65.07 | 0.98 | 2.85 | 0.09 | 0.25 | 20.48 | 24.81 | 72.21 |
| 7 | 321 | 48.30 | 5.25 | 84.55 | 1.67 | 3.76 | 0.12 | 0.26 | 26.82 | 26.59 | 60.00 |

TABLE V-B

| 1 Sample 6147-59 | 13 Area % AEEA | 14 % Selec. to AEEA | 15 Ratio: Obsv. DETA/ Obsv. PIP | 16 Area % of AEP | 17 % Selec. of AEP | 18 Area % NTEA | 19 % Selec. to NTEA | 20 % of NC | 21 Area % of TETA | 22 % Selec. of TETA | 23 Area % of DIAEP |
|---|---|---|---|---|---|---|---|---|---|---|---|
| 1 | 1.04 | 3.79 | 36.89 | 0.36 | 1.29 | 0.41 | 1.48 | 98.01 | 4.32 | 15.66 | 0.04 |
| 2 | 1.07 | 3.47 | 30.47 | 0.50 | 1.60 | 0.56 | 1.80 | 98.50 | 5.10 | 16.45 | 0.02 |
| 3 | 0.99 | 7.44 | 51.26 | 0.07 | 0.50 | 0.02 | 0.16 | 100.00 | 0.67 | 5.00 | 0.00 |
| 4 | 1.58 | 7.27 | 37.06 | 0.21 | 0.95 | 0.30 | 1.38 | 98.59 | 2.77 | 12.73 | 0.02 |
| 5 | 0.94 | 3.72 | 31.26 | 0.38 | 1.50 | 0.00 | 0.00 | 98.72 | 3.79 | 14.96 | 0.00 |
| 6 | 0.62 | 1.80 | 25.32 | 0.70 | 2.02 | 0.62 | 1.79 | 98.64 | 6.20 | 18.05 | 0.00 |
| 7 | 0.00 | 0.00 | 15.96 | 1.37 | 3.08 | 1.06 | 2.40 | 97.38 | 9.96 | 22.47 | 0.00 |

TABLE V-C

| 1 Sample 6147-59 | 24 % Selec. DIAEP | 25 Area % of PEEDA | 26 % Selec. PEEDA | 27 Area % of AETETA | 28 % Selec. AETETA | 29 Area % of TEPA | 30 % Selec. TEPA | 31 Area % Cycl N5s(1) | 32 % Sel. Cycl N5s(1) | 33 Area % PEHA+(2) | 34 % Selec. PEHA+(2) | 35 Total Product |
|---|---|---|---|---|---|---|---|---|---|---|---|---|
| 1 | 0.15 | 0.05 | 0.20 | 0.07 | 0.25 | 0.07 | 0.25 | 0.10 | 0.36 | 0.07 | 0.24 | 27.58 |
| 2 | 0.05 | 0.07 | 0.22 | 0.08 | 0.24 | 0.29 | 0.93 | 0.07 | 0.22 | 0.09 | 0.28 | 30.98 |
| 3 | 0.00 | 0.00 | 0.00 | 0.00 | 0.00 | 0.04 | 0.28 | 0.02 | 0.13 | 0.13 | 0.96 | 13.29 |
| 4 | 0.08 | 0.03 | 0.12 | 0.07 | 0.34 | 0.02 | 0.08 | 0.03 | 0.13 | 0.08 | 0.37 | 21.73 |
| 5 | 0.00 | 0.05 | 0.19 | 0.07 | 0.26 | 0.00 | 0.00 | 0.00 | 0.00 | 0.00 | 0.00 | 25.33 |
| 6 | 0.00 | 0.09 | 0.27 | 0.00 | 0.00 | 0.22 | 0.65 | 0.03 | 0.10 | 0.00 | 0.00 | 34.36 |
| 7 | 0.00 | 0.30 | 0.67 | 0.74 | 1.66 | 2.35 | 5.30 | 0.18 | 0.40 | 0.00 | 0.00 | 44.31 |

(1) Non-cyclic pentamines
(2) Pentaethylenehexamine and heavier products

The results reported in Table V are interesting in a number of respects. Note that at temperatures below about 300°C, such as the reaction temperature of 280°C used for Sample 3, the MEA conversion was only about 30%, but that the selectivity to non-cyclic products was excellent. At about 300°C (Sample No. 1 and Sample

No. 5), the MEA conversion was about 50% and the selectivity to non-cyclic products remained excellent at about 98 to 99%. As the temperature was increased still further (Sample No. 6 at 310°C and Sample No. 7 at 321°C) there was a significant increase in MEA conversion (about 65% for Sample No. 6 and about 85% for Sample No. 7) but there was no loss in the selectivity to non-cyclic products. This is the same phenomenon noted above in respect of Fig. 2. Also, note that at 321°C, the drop in selectivity to DETA to about 60% was more than compensated for by the increase in selectivity to TETA of about 22% and the unexpected production of a significant quantity of TEPA (selectivity about 5%), a product not produced to any significant extent at the lower temperatures.

## Claims

1. A catalyst composition comprising titania pellets containing fluorine and phosphorus and having from 0.01 to 5 wt.% of phosphorus thermally (chemically) bonded thereto, prepared by impregnating titania pellets with a solvent solution of a fluorophosphoric acid and thereafter drying and calcining the thus-treated titania pellets.

2. A catalyst composition according to Claim 1 characterised in that the titania pellets have from 0.1 to 2 wt.% of phosphorus thermally bonded thereto.

3. A method of preparing a catalyst composition according to Claim 1 or 2 characterised by immersing titania pellets in a solvent solution containing 10 to 50 wt.% of a fluorophosphoric acid for 0.1 to 5 hours at a temperature of 20 to 150°C and thereafter drying and calcining the thus-impregnated titania pellets at a temperature of 100 to 650°C for 2 to 24 hours.

4. A method according to Claim 3 characterised in that the fluorophosphoric acid is monofluorophosphoric acid or difluorophosphoric acid.

5. A method according to Claim 3 or 4 characterised in that the solvent is a polar organic solvent.

6. A method according to Claim 5 characterised in that the solvent is acetone.

7. Use of a catalyst according to Claim 1 or 2 are produced according to any one of Claims 3 to 6 in a process comprising continuously contacting a mixture of ethylenediamine and monoethanolamine in a molar ratio of 1 to 5 moles of ethylenediamine per mole of monoethanolamine with said pelleted catalyst at a temperature of 250 to 350°C and a pressure of 500 to 3000 psig (3.5 to 21 MPa) to obtain an essentially non-cyclic reaction product comprising polyethylene polyamines.

8. A use according to Claim 7 characterised in that the temperature is 270 to 320°C and the pressure is 1000 to 2000 psig (7 to 14 MPa).

## Patentansprüche

1. Katalysatorzusammensetzung, die Titanoxid-Pellets umfaßt, die Fluor und Phosphor enthalten und von 0,01 bis 5 Gew.-% Phosphor thermisch (chemisch) daran gebunden aufweisen, hergestellt durch Tränken von Titanoxid-Pellets mit einer Lösungsmittellösung einer Fluorophosphorsäure und anschließendes Trocknen und Kalzinieren der so behandelten Titanoxid-Pellets.

2. Katalysatorzusammensetzung nach Anspruch 1, dadurch gekennzeichnet, daß die Titanoxid-Pellets von 0,1 bis 2 Gew.-% Phosphor thermisch daran gebunden aufweisen.

3. Verfahren zur Herstellung einer Katalysatorzusammensetzung nach Anspruch 1 oder 2, gekennzeichnet durch Eintauchen von Titanoxid-Pellets in eine Lösungsmittelösung, die 10 bis 50 Gew.-% einer Fluorophosphorsäure enthält, für 0,1 bis 5 Stunden bei einer Temperatur von 20 bis 150°C und anschließendes Trocknen und Kalzinieren der so getränkten Titanoxid-Pellets bei einer Temperatur von 100 bis 650°C für 2 bis 24 Stunden.

4. Verfahren nach Anspruch 3, dadurch gekennzeichnet, daß die Fluorophosphorsäure eine

Monofluorophosphorsäure oder eine Difluorophosphorsäure ist.

5. Verfahren nach Anspruch 3 oder 4, dadurch gekennzeichnet, daß das Lösungsmittel ein polares organisches Lösungsmittel ist.

6. Verfahren nach Anspruch 5, dadurch gekennzeichnet, daß das Lösungsmittel Aceton ist.

7. Verwendung eines Katalysators nach Anspruch 1 oder 2, hergestellt nach einem der Ansprüche 3 bis 6, in einem Verfahren, das das kontinuierliche In-Kontakt-Bringen einer Mischung aus Ethylendiamin und Monoethanolamin in einem Molverhältnis von 1 zu 5 Mol Ethylendiamin pro Mol Monoethanolamin mit besagtem pelletiertem Katalysator bei einer Temperatur von 250 bis 350°C und einem Druck von 500 bis 3000 psig (3,5 bis 21 MPa) umfaßt, um ein im wesentlichen nicht-zyklisches Reaktionsprodukt zu erhalten, das Polyethylenpolyamine umfaßt.

8. Verwendung nach Anspruch 7, dadurch gekennzeichnet, daß die Temperatur 270 bis 320°C ist und daß der Druck 1000 bis 2000 psig (7 bis 14 MPa) ist.

## Revendications

1. Composition catalytique comprenant des particules d'oxyde de titane contenant du fluor et du phosphore, et comprenant de 0,01 à 5 % en poids de phosphore thermiquement (chimiquement) lié à celles-ci, préparée en imprégnant des particules d'oxyde de titane avec une solution d'un acide fluorophosphorique dans un solvant, et en séchant ensuite puis en calcinant les particules d'oxyde de titane ainsi traitées.

2. Composition catalytique selon la revendication 1, caractérisée en ce que les particules d'oxyde de titane comprennent de 0,1 à 2 % en poids de phosphore thermiquement lié à celles-ci.

3. Procédé de préparation d'une composition catalytique selon la revendication 1 ou 2, caractérisé en ce qu'on immerge des particules d'oxyde de titane dans une solution à base de solvant contenant de 10 à 50 % en poids d'un acide fluorophosphorique, pendant 0,1 à 5 h à une température de 20 à 150 °C, et ensuite on séche puis on calcine les particules d'oxyde de titane ainsi imprégnées, à une température de 100 à 650°C pendant 2 à 24h.

4. Procédé selon la revendication 3, caractérisé en ce que l'acide fluorophosphorique est l'acide monofluorophosphorique ou l'acide difluorophosphorique.

5. Procédé selon la revendication 3 ou 4, caractérisé en ce que le solvant est un solvant organique polaire.

6. Procédé selon la revendication 5, caractérisé en ce que le solvant est l'acétone.

7. Utilisation d'un catalyseur selon la revendication 1 ou 2 ou produit selon l'une quelconque des revendications 3 à 6, dans un procédé comprenant la mise en contact en continu d'un mélange d'éthylènediamine et de monoéthanolamine, selon un rapport molaire de 1 à 5 moles d'éthylènediamine par mole de monoéthanolamine, avec ledit catalyseur particulaire, à une température de 250 à 350 °C et sous une pression de 500 à 3000 psig (3,5 à 21 MPa) pour obtenir un produit de réaction pratiquement acyclique comprenant des polyéthylène-polyamines.

8. Utilisation selon la revendication 7, caractérisée en ce que la température est de 270 à 320 °C, et la pression de 1000 à 2000 psig (7 à 14 MPa).

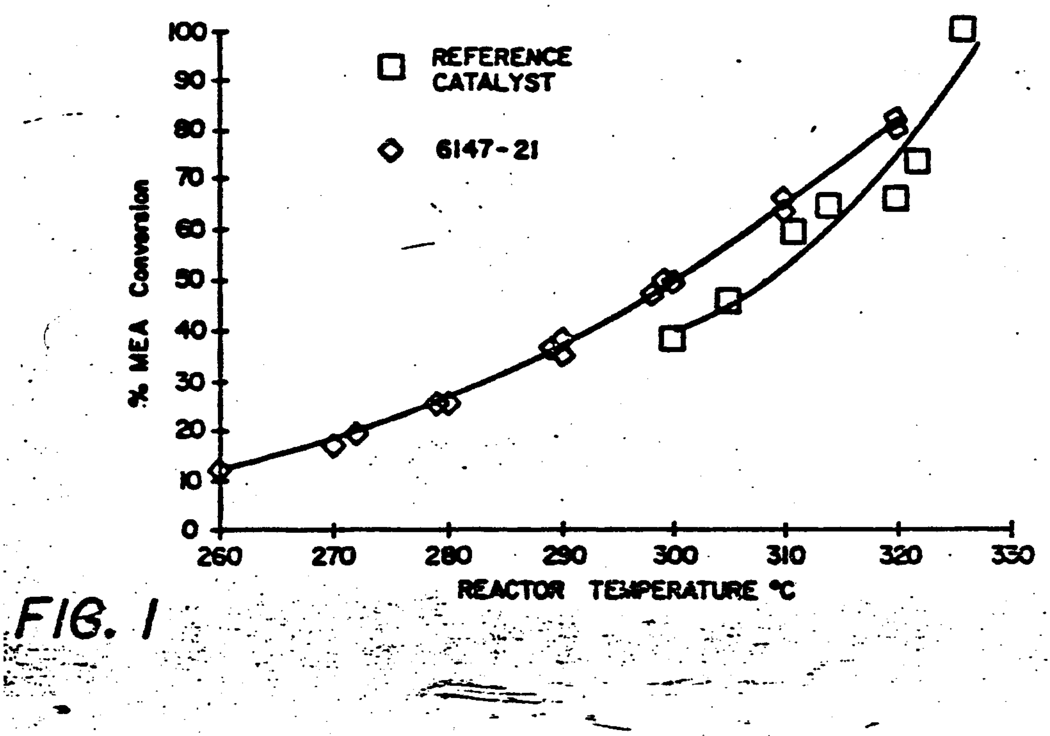
REFERENCE CATALYST
6147-21
% MEA Conversion
0
10
20
30
40
50
60
70
80
90
100
260
270
280
290
300
310
320
330
REACTOR TEMPERATURE °C

FIG. 1

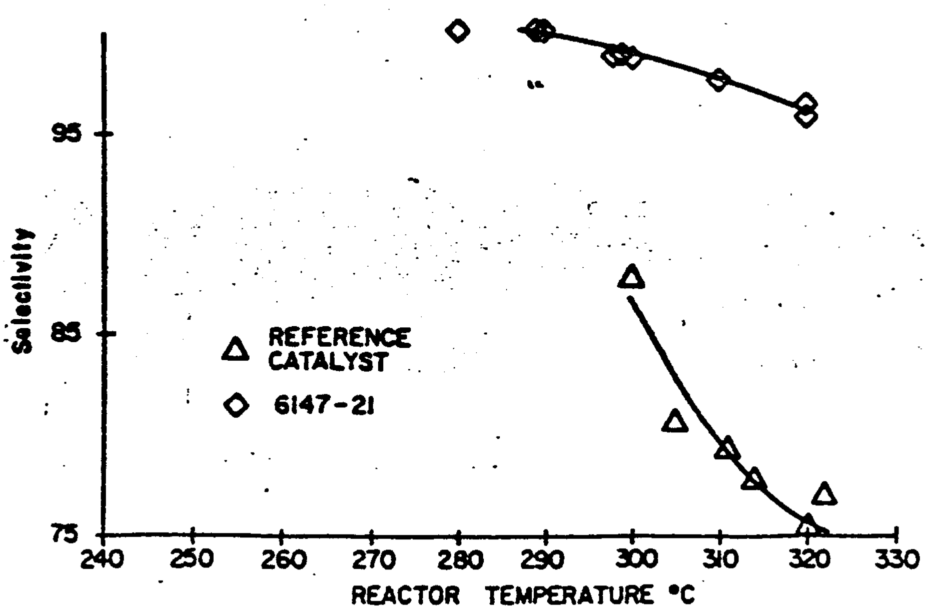
REFERENCE CATALYST
6147-21
Selectivity
75
85
95
240
250
260
270
280
290
300
310
320
330
REACTOR TEMPERATURE °C

FIG. 2